# EUROPÄISCHE PATENTSCHRIFT

(11) **EP 2 872 517 B1**
(45) Veröffentlichungstag und Bekanntmachung des Hinweises auf die Patenterteilung: **27.04.2016**
(21) Anmeldenummer: 13752816.2
(22) Anmeldetag: 11.07.2013
(51) Int. Cl.: C07D 311/94, C07D 493/06, C08K 5/1545

(54) **PHOTOCHROME FLUORENOPYRANE MIT DEFINIERTER DIBENZO[B,D]-PYRANO-ANNELLIERUNG**
PHOTOCHROMIC FLUORENOPYRANS WITH DEFINED DIBENZO[B,D]PYRANO FUSED ATTACHMENT
FLUORÉNOPYRANES PHOTOCHROMES PRÉSENTANT UNE ANNELLATION DIBENZO[B,D]-PYRANO DÉFINIE

(30) Priorität: 12.07.2012 DE 102012013748
(43) Veröffentlichungstag der Anmeldung: 20.05.2015
(73) Patentinhaber: Rodenstock GmbH, 80687 München (DE)
(72) Erfinder: WEIGAND, Udo, 81247 München (DE); ROHLFING, Yven, 81547 München (DE); ZINNER, Herbert, 85296 Rohrbach (DE)
(74) Vertreter: Müller-Boré & Partner Patentanwälte PartG mbB
(86) Internationale Anmeldenummer: PCT/EP2013/002065
(87) Internationale Veröffentlichungsnummer: WO 2014/009020

(56) Entgegenhaltungen:
- EP-A1- 0 912 908
- US-A- 5 645 767
- US-A- 5 698 141

## Beschreibung

Die vorliegende Erfindung betrifft photochrome Fluorenopyrane mit definierter Dibenzo[b,d]-pyrano-Annellierung gemäß den allgemeinen Formeln (I) oder (II) und deren Verwendung in Kunststoffen aller Art, insbesondere für ophthalmische Zwecke. Die erfindungsgemäßen photochromen Verbindungen zeichnen sich durch zwei ausgeprägte Absorptionsbanden der offenen Form im sichtbaren Wellenlängenbereich aus, d.h. mit derartigen Farbstoffmolekülen lassen sich zwei herkömmliche photochrome Farbstoffe, die jeweils nur eine diskrete Absorptionsbande aufweisen, ersetzen. Die erfindungsgemäßen Verbindungen weisen zudem eine sehr gute Lebensdauer bei sehr hoher Leistung auf.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, dass diese Farbstoffmoleküle durch Lichtenergie in einen angeregten Zustand ("offene Form") übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, deren offene, angeregte Formen verschiedene Eindunkelungsfarben von Gelb bis Rotviolett zeigen.

2,2-Diaryl-2H-naphtho[1,2-b]pyrane mit einer zusätzlichen Annellierung am pyrano-annellierten Benzolring sind von großem Interesse, da sie aufgrund ihres größeren Ringsystems längerwellig absorbieren und so violette bzw. blaue Eindunklungsfarben zugänglich sind. Bei der Annellierung handelt es sich um einen substituierten Benzolring (in der Formel (I) bzw. (II) der hierin beschriebenen erfindungsgemäßen Verbindungen demgemäß der Benzolring mit den Substituenten R₃), der nochmals in ortho-Stellung mit dem Naphthopyran verbrückt ist.

Wird diese Verbrückung nur über ein Atom hergestellt, so ergibt sich ein an das Naphthopyran annellierter Fünfring. Die Verwendung von Heteroatomen, insbesondere Sauerstoff, als Brücke wird in US 5,651,923 sowie US 6,018,059 beschrieben. Mit Kohlenstoff als Brückenatom ("einfach-indenoannellierte Naphthopyrane") existieren eine Reihe von Patentanmeldungen (z.B. EP 0 792 468, EP 0 906 366, EP 0 987 260, EP 1 054 010, EP 1 116 723 sowie EP 1 184 379), die sich vor allem hinsichtlich der beiden Substituenten am Kohlenstoff-Brückenatom unterscheiden. Diese Substituenten haben einen großen Einfluss auf die Aufhellgeschwindigkeit der offenen (angeregten) Form. Die offenen Formen all dieser photochromen Farbstoffe, die zusätzlich noch Substituenten wie Alkyl oder Alkoxy am nicht-indenoannellierten Benzolring der Naphthopyran-Einheit aufweisen können, weisen jeweils keine Doppelabsorptionsbande im sichtbaren Wellenlängenbereich auf.

In EP 1 674 460 sowie WO 2011/034202 werden einfach-indenoannellierte Naphthopyrane offenbart, die zusätzlich einen Aryl-Substituenten am nicht-indenoannellierten Benzolring der Naphthopyran-Einheit aufweisen.

In EP 0 912 908 sowie EP 0 958 514 werden indenoannellierte Naphthopyrane offenbart, die zusätzlich noch eine heterocyclische Annellierung (z.B. Benzothiophen-, Benzofuran- oder Indol-Ringsystem) am nicht-indenoannellierten Benzolring der Naphthopyran-Einheit aufweisen. Diese Verbindungen weisen jedoch keinen Doppelabsorptionscharakter auf.

Wird diese Verbrückung über zwei Atome erzeugt, so ergibt sich ein annellierter Sechsring mit allein für C, O und N vielfältigen Möglichkeiten. Verbindungen mit C=O und N-R (Lactam-Brücke) werden in US 6,379,591 beschrieben. Verbindungen mit einer unsubstituierten CH₂-CH₂-Brücke sowie einem weiteren annellierten Heterocyclus in 7,8-Stellung des zugrundeliegenden Benzopyrans sind in US 6,426,023 offenbart. US 6,506,538 beschreibt die carbocyclischen Analogverbindungen, bei denen die H-Atome in der Brücke durch OH, (C₁-C₆) Alkoxy oder zwei H-Atome an einem C-Atom durch =O ersetzt sein können. US 6,022,495 beschreibt *inter alia* Verbindungen mit einer O-CR¹R²-Brücke.

Wird diese Verbindung durch drei Atome erzeugt, ergibt sich ein annellierter Siebenring mit sehr vielen Variationsmöglichkeiten durch Einfügen von Heteroatomen. Verbindungen mit einer CH₂-CH₂-CH₂-Brücke sind in US 6,558,583 beschrieben. Auch hier können die H-Atome in der Brücke durch OH, (C₁-C₆)-Alkyl oder (C₁-C₆)-Alkoxy oder zwei Wasserstoffatome an einem C-Atom durch =O ersetzt sein. Die offene Form dieser photochromen Farbstoffe absorbiert bei gleichem Substitutionsmuster kürzerwellig als die annellierten Fünf- und Sechsringe.

Die verschiedenen, im Stand der Technik verfügbaren photochromen Farbstoffe haben jedoch Nachteile, die bei der Verwendung in Sonnenschutzgläsern den Tragekomfort des Brillenträgers wesentlich beeinträchtigen. Zum einen weisen die Farbstoffe eine nicht ausreichend langwellige Absorption im angeregten wie im nicht angeregten Zustand auf. Zum anderen liegt häufig eine zu hohe Temperaturempfindlichkeit der Eindunkelung vor, wobei gleichzeitig eine zu langsame Aufhellung eintreten kann. Darüber hinaus besitzen die im Stand der Technik verfügbaren Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Den vorstehenden, im Stand der Technik verfügbaren photochromen Farbstoffen ist gemeinsam, dass sie nur eine Absorptionsbande der offenen Form im sichtbaren Wellenlängenbereich zeigen. Um in Neutralfarben - d.h. in Grau- oder Brauntönen - eindunkelnde phototrope Gläser zu realisieren, ist insofern ein Abstimmungsprozeß zwischen den verschiedenen photochromen Farbstoffen einer Mischung hinsichtlich Aufhellungsgeschwindigkeit, Lebensdauer sowie spektralen Anregungseigenschaften erforderlich, damit das phototrope Glas zu jedem Zeitpunkt des Eindunklungs- und Aufhellungscylus denselben Farbton aufweist. Es wäre daher äußerst wünschenswert, auf diesen Abstimmungsprozeß verzichten zu können.

Der vorliegenden Erfindung liegt daher die Aufgabe zugrunde, photochrome Farbstoffe bereitzustellen, mit denen es möglich ist, in Neutralfarben - d.h. in Grau- oder Brauntönen - eindunkelnde phototrope Gläser mit nur einem solchen photochromen Farbstoff zu realisieren. Solche photochrome Farbstoffe sollen sich zudem durch die Kombination von langwelligem Absorptionsmaximum der geschlossenen Form mit steiler Kante zum sichtbaren Wellenlängenbereich, hoher Eindunkelungsleistung, sehr schneller Aufhellreaktion und sehr guter Lichtbeständigkeit auszeichnen.

Diese Aufgabe wird durch die in den Ansprüchen gekennzeichneten Gegenstände gelöst.

Insbesondere werden photochrome Fluorenopyrane mit definierter Dibenzo[b,d]-pyrano-Annellierung gemäß den allgemeinen Formeln (I) oder (II) bereitgestellt: 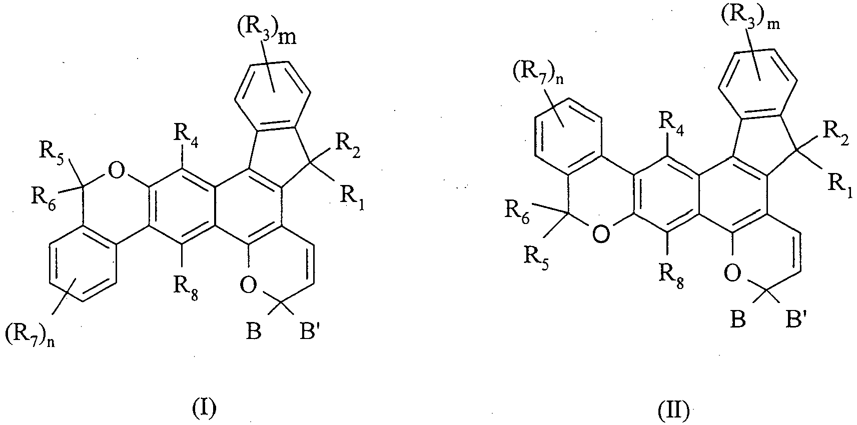
wobei die Reste R₁, R₂, R₃, R₄, R₅, R₆, R₇ und R₈ jeweils unabhängig voneinander einen Substituenten darstellen, ausgewählt aus der Gruppe α, bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome, wie beispielsweise O oder S, aufweisen kann, einem (C₁-C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α, vorzugsweise aus (C₁-C₆)-Alkyl, (C₁-C₆)-Alkoxy, Brom, Chlor oder Fluor, ausgewählt sein können;
m und n unabhängig voneinander eine ganze Zahl von 1 bis 4 darstellen,
oder die Reste R₁ und R₂ zusammen mit dem an diese Reste gebundenen Kohlenstoffatom einen 3- bis 8-gliedrigen, vorzugsweise 5- bis 7-gliedrigen, carbo- oder heterocyclischen (d.h. Sauerstoff- oder Schwefelatome-haltige Heterocyclen) Ring bilden, der gegebenenfalls einen oder mehrere, vorzugsweise einen bis vier, Substituenten aus der Gruppe α trägt, wobei an diesen Ring aber auch ein bis drei aromatische oder heteroaromatische Ringsysteme annelliert sein können, wobei das bzw. die Ringsysteme unabhängig voneinander aus der Gruppe β ausgewählt ist bzw. sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, und wobei, wenn zwei dieser am 3- bis 8-gliedrigen carbo- oder heterocyclischen Ring getragenen Substituenten am selben Ringkohlenstoffatom sitzen, diese wiederum einen 3- bis 8-gliedrigen carbo- oder heterocyclischen Ring bilden können,
oder die Reste R₅ und R₆ zusammen mit dem an diese Reste gebundenen Kohlenstoffatom einen 3- bis 8-gliedrigen, vorzugsweise 5- bis 7-gliedrigen, carbo- oder heterocyclischen (d.h. Sauerstoff- oder Schwefelatome-haltige Heterocyclen) Ring bilden, der gegebenenfalls einen oder mehrere, vorzugsweise einen bis vier, Substituenten aus der Gruppe α trägt, wobei an diesen Ring aber auch ein bis drei aromatische oder heteroaromatische Ringsysteme annelliert sein können, wobei das bzw. die Ringsysteme unabhängig voneinander aus der Gruppe β ausgewählt ist bzw. sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, und wobei, wenn zwei dieser am 3- bis 8-gliedrigen carbo- oder heterocyclischen Ring getragenen Substituenten am selben Ringkohlenstoffatom sitzen, diese wiederum einen 3- bis 8-gliedrigen carbo- oder heterocyclischen Ring bilden können,
oder zwei benachbarte Reste R₃ einen annellierten Benzol-Ring bilden, der un-, mono- oder disubstituiert sein kann, wobei die Substituenten wiederum aus der Gruppe α ausgewählt sein können;
oder zwei benachbarte Reste R₇ einen annellierten Benzol-Ring bilden, der un-, mono- oder disubstituiert sein kann, wobei die Substituenten wiederum aus der Gruppe α ausgewählt sein können;
und B und B' unabhängig voneinander aus einer der folgenden Gruppen a) oder b) ausgewählt sind, wobei
   a) mono-, di- und trisubstituierte Arylreste sind, wobei der Arylrest Phenyl, Naphthyl oder Phenanthryl ist;
   b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist,
wobei die Substituenten der Aryl- oder Heteroarylreste in a) und b) solche sind, ausgewählt aus der vorstehend definierten Gruppe α oder der Gruppe χ, bestehend aus Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁-C₆)-Alkyl, (C₁-C₆)-Alkoxy, Brom, Chlor oder Fluor ausgewählt sein können;
oder wobei zwei direkt benachbarte Substituenten der Aryl- oder Heteroarylreste in a) und b) eine V-(CR₈R₉)ₚ-W-Gruppierung darstellen, wobei p = 1, 2 oder 3 ist, die Reste R₈ und R₉ jeweils unabhängig voneinander einen Substituenten darstellen, ausgewählt aus der Gruppe α, und wobei V und W unabhängig voneinander -O-, -S-, -N(C₁-C₆)Alkyl, -NC₆H₅, -CH₂-, -C(CH₃)₂- oder -C(C₆H₅)₂- sein können, wobei zwei oder mehrere benachbarte CR₈R₉-Einheiten dieser V-(CR₈R₉)ₚ-W-Gruppierung Teil eines daran annellierten Benzolrings sein können, welcher jeweils wiederum einen oder mehrere Substituenten, ausgewählt aus der Gruppe α aufweisen kann, oder V und/oder W zusammen mit der jeweils benachbarten CR₈R₉ Einheit einen annellierten Benzolring, der un-, mono- oder disubstituiert sein kann, wobei dessen Substituenten aus der Gruppe α ausgewählt sein können, darstellen.

Die Molekülstruktur der erfindungsgemäßen Verbindungen weist ein zugrundeliegendes Fluoren-Ringsystem mit den Substituenten R₁ bis R₃ auf. An einem Benzolring dieses Fluorens ist sowohl eine Dibenzo[b,d]-Annellierung mit den Substituenten R₄ bis R₈ als auch die photolabile Pyran-Einheit mit den Substituenten B und B' gebunden. Letztere ist für den photochromen Charakter verantwortlich, da durch Anregung mit langwelligem UVA-Licht die Bindung zwischen dem Sauerstoff der Pyran-Einheit und dem Kohlenstoffatom mit den Substituenten B und B' reversibel gebrochen wird, wodurch ein farbiges Merocyanin-System entsteht.

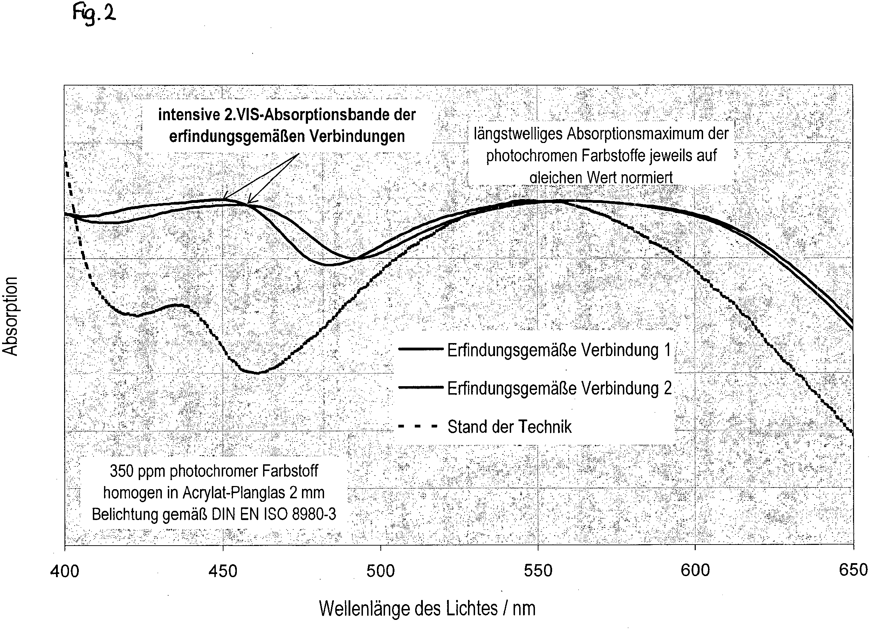

Die erfindungsgemäßen Verbindungen zeichnen sich gegenüber den im Stand der Technik (US 5,645,767) bekannten photochromen 2H-Naphtho[1,2-b]pyranen, die keine Dibenzo[b,d]-pyrano-Annellierung aufweisen, dadurch aus, dass sie eine Doppelabsorptionsbande, d.h. zwei starke Absorptionsbanden, der offenen Form im sichtbaren Wellenlängenbereich zeigen; vgl. Figur 2.

Die erste der beiden starken Absorptionsbanden weist dabei ein Absorptionsmaximum von > 500 nm auf, während das Maximum der zweiten Bande im kürzerwelligen sichtbaren Bereich (400-500 nm) liegt. Aufgrund letzterer Bande ist es mit den erfindungsgemäßen Verbindungen möglich, auf gelb- oder orange-eindunkelnde photochrome Farbstoffe in neutralfarbenen phototropen Gläsern zu verzichten. Dies ist einerseits wichtig für Polymersysteme, in denen diese gelb- und orange-eindunkelnden Farbstoffe - aufgrund ihrer anderen Molekülstruktur im Vergleich zu den längerwellig absorbierenden violett- und blaueindunkelnden Farbstoffen - eine ungenügende Lebensdauer aufweisen oder andere Nachteile mit sich bringen. Andererseits ist es mit den erfindungsgemäßen photochromen Farbstoffen erstmals möglich, in Neutralfaben - d.h. in Grau- oder Brauntönen - eindunkelnde phototrope Gläser mit nur einem photochromen Farbstoff zu realisieren. Damit entfällt der bisher notwendige mühsame Abstimmungsprozess zwischen den verschiedenen photochromen Farbstoffen einer Mischung hinsichtlich Aufhellungsgeschwindigkeit, Lebensdauer sowie spektralen Anregungseigenschaften, damit das phototrope Glas zu jedem Zeitpunkt des Eindunklungs- und Aufhellungscylus denselben Farbton aufweist.

Da die erfindungsgemäßen Verbindungen darüber hinaus hohe Augenklarheit (d.h. hohe Transmission im nicht angeregten Zustand) sowie sehr gute Lichtbeständigkeit aufweisen, sind sie hervorragend geeignet zum Einsatz in phototropen Gläsern.

Darüber hinaus sind die erfindungsgemäßen Verbindungen im nicht angeregten Zustand völlig farblos (d.h. ohne kosmetisch störenden Gelbton, da die Absorption der geschlossenen Form auf den UV-Bereich beschränkt ist) und weisen eine sehr gute Lebensdauer auf.

Die vorliegenden Erfindung liegt *inter alia* die Erkenntnis zugrunde, dass sich durch eine genau definierte Dibenzo[b,d]pyrano-Annellierung mit den Substituenten R₄ bis R₈ an ein Fluorenopyran-System eine ausgeprägte Doppelabsorptionsbande der offenen Form im sichtbaren Wellenlängenbereich generieren lässt. Im Gegensatz zum vorstehend beschriebenen Stand der Technik ist in den erfindungsgemäßen Verbindungen erstmals ein Benzolring mit den Substituenten R₇ mittels einer speziellen Verbrückung (-O-CR₅R₆-) an den photochromen Molekülrest gebunden. Diese erfindungsgemäße spezielle Verbrückung bewirkt zwei positive Effekte: Zum einen richtet sie den Benzolring mit den Substituenten R₇ parallel zur Ebene des Restmoleküls aus, was eine optimale Überlappung der π-Etektronen und somit eine entsprechend längerwellige Absorption bewirkt. Zum anderen sorgt sie noch für einen zusätzlichen Donor-Effekt (ähnlich einer Methoxygruppe). Die Kombination dieser beiden Effekte ist bisher nicht im Stand der Technik beschrieben und ist für die intensiven und längerwellig verschobenen Doppelabsorptions-Banden der offenen Form der erfindungsgemäßen Verbindungen verantwortlich. Aufgrund dieser Doppelabsorptionsbande im sichtbaren Wellenlängenbereich lassen sich mit einem derartigen erfindungsgemäßen Molekül zwei konventionelle photochrome Farbstoffe - mit jeweils einer diskreten Absorptionsbande - ersetzen.
Fig. 1 zeigt ein entsprechendes Syntheseschema zur Herstellung der erfindungsgemäßen Verbindungen gemäß der vorstehenden Formel (I).
Fig. 2 zeigt die UV-Absorptionsspektren spezifischer erfindungsgemäßer Verbindung im Vergleich zum Stand der Technik.

In einer Ausführungsform der vorliegenden Erfindung sind die Reste R₁ und R₂ unabhängig voneinander aus einem Wasserstoffatom, einem (C₁-C₆)-Alkylrest oder einem (C₃-C₇)-Cycloalkylrest, vorzugsweise einem (C₁-C₆)-Alkylrest oder einem (C₃-C₇)-Cycloalkylrest, ausgewählt.

In einer anderen Ausführungsform der vorliegenden Erfindung bilden die Reste R₁ und R₂ zusammen mit dem an diese Reste gebundenen Kohlenstoffatom einen 5-bis 7-gliedrigen carbo- oder heterocyclischen Ring, der gegebenenfalls einen oder mehrere Substituenten aus der Gruppe α trägt.

In einer weiteren Ausführungsform der vorliegenden Erfindung sind die Reste R₅ und R₆ unabhängig voneinander aus einem Wasserstoffatom, einem (C₁-C₆)-Alkylrest oder einem (C₃-C₇)-Cycloalkylrest, vorzugsweise einem Wasserstoffatom oder einem (C₁-C₆)-Alkylrest, ausgewählt.

Bevorzugte photochrome Fluorenopyrane mit definierter Dibenzo[b,d]-pyrano-Annellierung gemäß der vorliegenden Erfindung weisen die Formel (I) auf.

In einer weiter bevorzugten Ausführungsform der vorliegenden Erfindung weisen die photochromen Fluorenopyrane mit definierter Dibenzo[b,d]-pyrano-Annellierung die nachstehende Formel (III) auf. 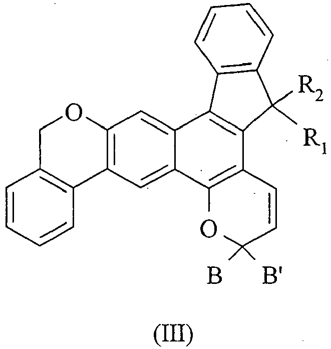 wobei die Reste R₁, R₂, B sowie B' wie vorstehend definiert sind.

In einer weiteren bevorzugten Ausführungsform sind die Reste B und B' unabhängig voneinander aus der Gruppe a), wie vorstehend definiert, ausgewählt.

Die Substituenten der Gruppe χ, welche Stickstoffatome aufweisen bzw. Amingruppen tragen, sind über diese an den Phenyl-, Naphthyl- bzw. Phenanthrylrest der Gruppe a) gebunden.

Wenn bezüglich der Substituenten der Gruppe V-(CR₈R₉)ₚ-W-Gruppierung, welche an den Phenyl, Naphthyl- bzw. Phenanthrylrest der Gruppe a) für die Reste B bzw. B' gebunden sein können, zwei oder mehrere benachbarte Kohlenstoffatome dieser V-(CR₈R₉)ₚ-W-Gruppierung jeweils unabhängig voneinander Teil eines daran annellierten Benzo-Ringsystems sind, so bedeutet dies, dass dann die beiden Methylenkohlenstoffatome (-CH₂-CH₂-) Teil eines annellierten Ringsystems werden. Wenn beispielsweise zwei oder drei Benzoringe annelliert sind, so können beispielsweise hier dann folgende Struktureinheiten vorliegen, wie nachstehend angeführt.

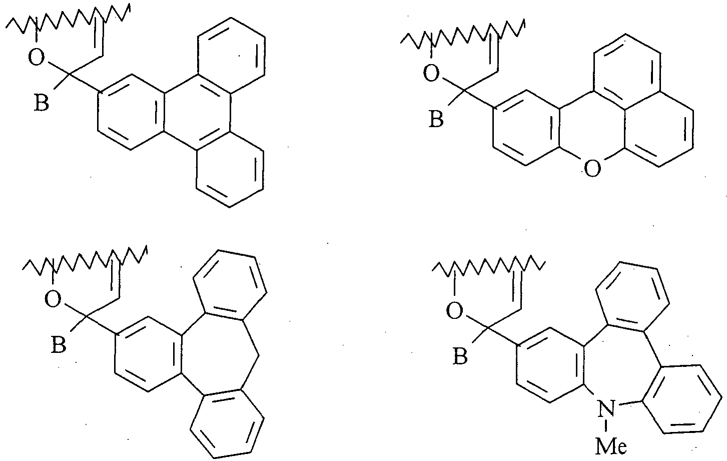

Selbstverständlich kann aber auch nur ein, über zwei benachbarte Kohlenstoffatome dieser V-(CR₈R₉)ₚ-W-Gruppierung annellierter Benzoring vorliegen.

Wie bereits ausgeführt, weisen die erfindungsgemäßen Verbindungen gegenüber den im Stand der Technik (US 5,645,767) bekannten photochromen 2H-Naphtho[1,2-b]pyranen, die keine Dibenzo[b,d]-pyrano-Annellierung aufweisen, überraschenderweise eine zweite starke Absorptionsbande der offenen Form im sichtbaren Wellenlängenbereich auf (siehe Figur 2). Die Ausbildung dieser zweiten Absorptionsbande bei den erfindungsgemäßen Verbindungen ist insofern unerwartet.

Zur Messung der spektralen Eigenschaften der erfindungsgemäßen Verbindungen wurden jeweils 350 ppm des photochromen Farbstoffes in einer Acrylatmonomer-Matrix gelöst und nach Zusatz eines Polymerisations-Initiators mit Hilfe eines Temperaturprogrammes thermisch polymerisiert. Die Transmissionseigenschaften im angeregten Zustand der so hergestellten Kunststoffgläser (Dicke 2 mm) wurden anschließend gemäß DIN EN ISO 8980-3 gemessen.

Die Strukturen der in Figur 2 eingesetzten bzw. untersuchten Verbindungen sind aus der folgenden Tabelle ersichtlich:

**Tabelle 1: Tabellarischer Vergleich der längstwelligen Absorptionsmaxima im angeregten Zustand (An = Anisyl, d.h. der 4-Methoxyphenyl-Rest)**

| | | | | |
|---|---|---|---|---|
| 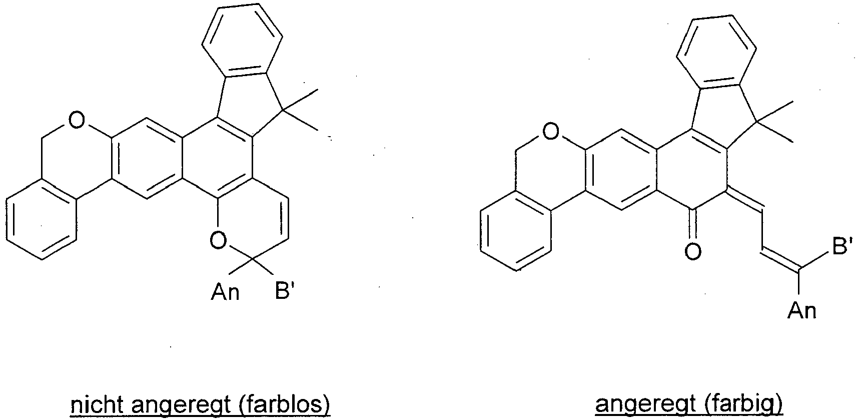 | | | | |

| | B' | λmax (1) | λmax (2) | Farbeindruck |
|---|---|---|---|---|
| Stand der Technik aus US 5,645,767 (ohne die erfindungsgemäße Dibenzo[b,d]-pyrano-Annellierung) | Anisyl | - | 550 nm | Violett |
| Erfindungsgemäße Verbindung 1 | Anisyl | 450 nm | 560 nm | (rötliches) Grau |
| Erfindungsgemäße Verbindung 2 | 6-Methoxy-2-naphthyl | 455 nm | 565 nm | (bräunliches) Grau |

Fig. 2 zeigt die UV-Absorptionsspektren der erfindungsgemäßen Verbindungen 1 und 2 im Vergleich zum Stand der Technik. Die Ausbildung einer Doppelabsorptionsbande bei den erfindungsgemäßen Verbindungen im Gegensatz zum Stand der Technik zeigt deutlich den Einfluss der Dibenzo[b,d]-pyrano-Annellierung auf das Absorptionsspektrum - bei ansonsten gleicher Molekülstruktur (siehe Figur 2).

Durch die vorliegende Erfindung wird eine Klasse neuer photochromer Doppelabsorptionsfarbstoffe bereitgestellt, die - je nach Wahl der Substituenten R₁ und R₂ - sowohl Verbindungen mit extrem tiefer Eindunklung und langsamer Aufhellgeschwindigkeit (für phototrope Outdoor-Produkte und höhere Temperaturen) als auch Verbindungen mit schnellerer Aufhellgeschwindigkeit (für phototrope Alltagsgläser) enthält.

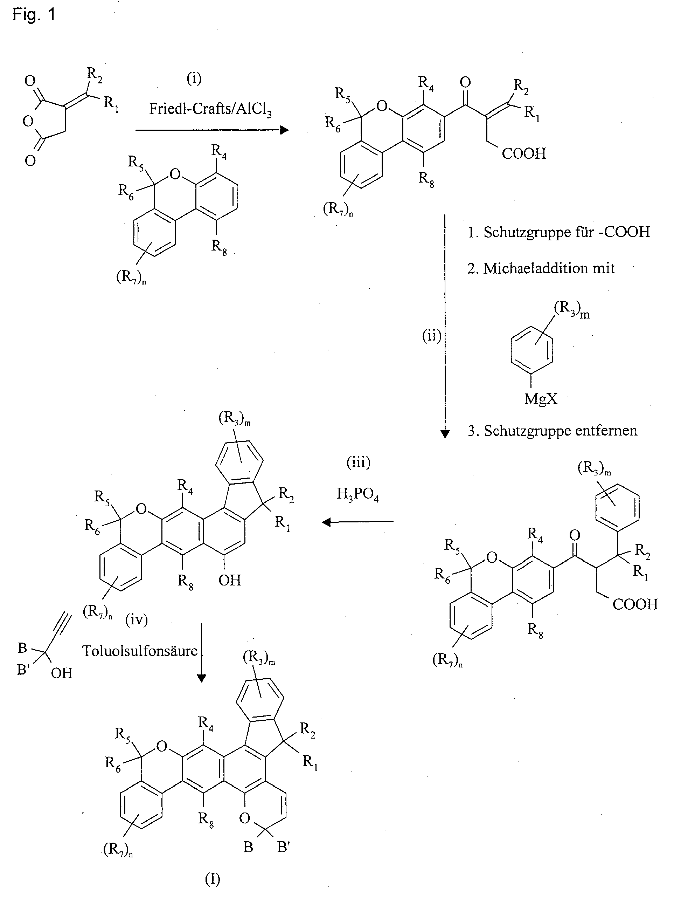

Zur Synthese der erfindungsgemäßen Verbindungen werden geeignet substituierte Methylidensuccinanhydride in einem ersten Schritt einer Friedel-Crafts-Reaktion mit geeignet substituierten Dibenzo[b,d]-pyrano-Derivaten unterworfen (Schritt (i)). Die -COOH Gruppe des daraus resultierenden Intermediats wird anschließend geschützt und dieses Intermediat einer Michael-Addition mit entsprechend substituierten Aryl-Grignardverbindungen unterworfen (Schritt (ii)). Nach Entfernung der Carbonsäure-Schutzgruppe werden *via* intramolekularer Cyclisierung mittels Phosphorsäure entsprechend substituierte Derivate gebildet (Schritt (iii)). Anschließend werden diese substituierten Derivate mit geeignet substituierten 2-Propin-1-ol-Derivaten gemäß Schritt (iv) zu den erfindungsgemäßen Verbindungen umgesetzt. Das vorstehende Syntheseschema ist in Figur 1 wiedergegeben. Zur Herstellung von erfindungsgemäßen Verbindungen mit der Formel (II) werden im Zuge der Friedel-Crafts-Reaktion die entsprechenden strukturisomeren Dibenzopyrano-Derivate eingesetzt.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Fluorenopyran-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen und dergleichen eingesetzt werden. Ferner können die erfindungsgemäßen photochromen Fluorenopyrane mit definierter Dibenzo[b,d]-pyrano-Annellierung beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Fluorenopyrane mit definierter Dibenzo[b,d]-pyrano-Annellierung durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Fluorenopyrane mit definierter Dibenzo[b,d]-pyrano-Annellierung beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

## Patentansprüche

1. Photochrome Fluorenopyrane mit definierter Dibenzo[b,d]-pyrano-Annellierung gemäß den allgemeinen Formeln (I) oder (II): 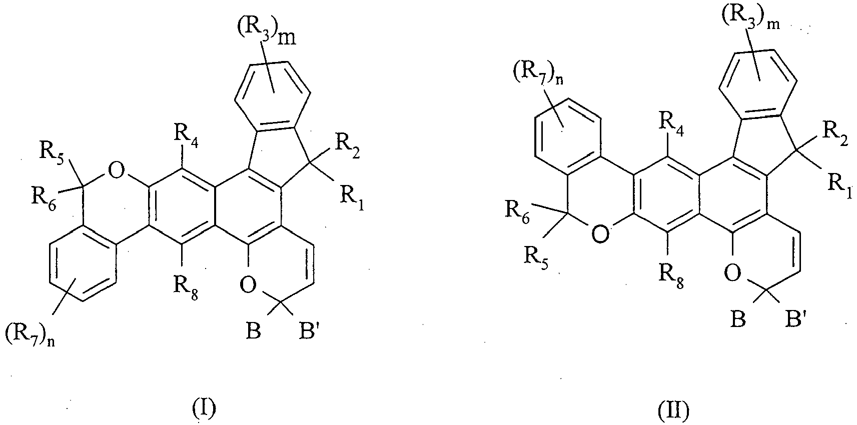
wobei die Reste R₁, R₂, R₃, R₄, R₅ ,R₆, R₇ und R₈ jeweils unabhängig voneinander einen Substituenten darstellen, ausgewählt aus der Gruppe α, bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome, wie beispielsweise O oder S, aufweisen kann, einem (C₁-C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α, vorzugsweise aus (C₁-C₆)-Alkyl, (C₁-C₆)-Alkoxy, Brom, Chlor oder Fluor, ausgewählt sein können;
m und n unabhängig voneinander eine ganze Zahl von 1 bis 4 darstellen,
oder die Reste R₁ und R₂ zusammen mit dem an diese Reste gebundenen Kohlenstoffatom einen 3- bis 8-gliedrigen, carbo- oder heterocyclischen Ring bilden, der gegebenenfalls einen oder mehrere, vorzugsweise einen bis vier, Substituenten aus der Gruppe α trägt, wobei an diesen Ring aber auch ein bis drei aromatische oder heteroaromatische Ringsysteme annelliert sein können, wobei das bzw. die Ringsysteme unabhängig voneinander aus der Gruppe β ausgewählt ist bzw. sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, und wobei, wenn zwei dieser am 3- bis 8-gliedrigen carbo- oder heterocyclischen Ring getragenen Substituenten am selben Ringkohlenstoffatom sitzen, diese wiederum einen 3- bis 8-gliedrigen carbo- oder heterocyclischen Ring bilden können,
oder die Reste R₅ und R₆ zusammen mit dem an diese Reste gebundenen Kohlenstoffatom einen 3- bis 8-gliedrigen, carbo- oder heterocyclischen Ring bilden, der gegebenenfalls einen oder mehrere, vorzugsweise einen bis vier, Substituenten aus der Gruppe α trägt, wobei an diesen Ring aber auch ein bis drei aromatische oder heteroaromatische Ringsysteme annelliert sein können, wobei das bzw. die Ringsysteme unabhängig voneinander aus der Gruppe β ausgewählt ist bzw. sind, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten, ausgewählt aus der Gruppe α, substituiert sein können, und wobei, wenn zwei dieser am 3- bis 8-gliedrigen carbo- oder heterocyclischen Ring getragenen Substituenten am selben Ringkohlenstoffatom sitzen, diese wiederum einen 3- bis 8-gliedrigen carbo- oder heterocyclischen Ring bilden können,
oder zwei benachbarte Reste R₃ einen annellierten Benzol-Ring bilden, der un-, mono- oder disubstituiert sein kann, wobei die Substituenten wiederum aus der Gruppe α ausgewählt sein können;
oder zwei benachbarte Reste R₇ einen annellierten Benzol-Ring bilden, der un-, mono- oder disubstituiert sein kann, wobei die Substituenten wiederum aus der Gruppe α ausgewählt sein können;
und B und B' unabhängig voneinander aus einer der folgenden Gruppen a) oder b) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist,
wobei die Substituenten der Aryl- oder Heteroarylreste in a) und b) solche sind, ausgewählt aus der vorstehend definierten Gruppe α oder der Gruppe χ, bestehend aus Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁-C₆)-Alkyl, (C₁-C₆)-Alkoxy, Brom, Chlor oder Fluor ausgewählt sein können;
oder wobei zwei direkt benachbarte Substituenten der Aryl- oder Heteroarylreste in a) und b) eine V-(CR₈R₉)ₚ-W-Gruppierung darstellen, wobei p = 1, 2 oder 3 ist, die Reste R₈ und R₉ jeweils unabhängig voneinander einen Substituenten darstellen, ausgewählt aus der Gruppe α, und wobei V und W unabhängig voneinander -O-, -S-, -N(C₁-C₆)Alkyl, -NC₆H₅, -CH₂-, -C(CH₃)₂- oder -C(C₆H₅)₂- sein können, wobei zwei oder mehrere benachbarte CR₈R₉-Einheiten dieser V-(CR₈R₉)ₚ-W-Gruppierung Teil eines daran annellierten Benzolrings sein können, welcher jeweils wiederum einen oder mehrere Substituenten, ausgewählt aus der Gruppe α aufweisen kann, oder V und/oder W zusammen mit der jeweils benachbarten CR₈R₉ Einheit einen annellierten Benzolring, der un-, mono- oder disubstituiert sein kann, wobei dessen Substituenten aus der Gruppe α ausgewählt sein können, darstellen.

2. Photochrome Fluorenopyrane mit definierter Dibenzo[b,d]-pyrano-Annellierung gemäß Anspruch 1, wobei die Reste R₁ und R₂ unabhängig voneinander aus einem Wasserstoffatom, einem (C₁-C₆)-Alkylrest oder einem (C₃-C₇)-Cycloalkylrest, vorzugsweise einem (C₁-C₆)-Alkylrest oder einem (C₃-C₇)-Cycloalkylrest, ausgewählt sind.

3. Photochrome Fluorenopyrane mit definierter Dibenzo[b,d]-pyrano-Annellierung gemäß Anspruch 1, wobei R₁ und R₂ zusammen mit dem an diese Reste gebundenen Kohlenstoffatom einen 5- bis 7-gliedrigen carbo- oder heterocyclischen Ring bilden, der gegebenenfalls einen oder mehrere Substituenten aus der Gruppe α trägt.

4. Photochrome Fluorenopyrane mit definierter Dibenzo[b,d]-pyrano-Annellierung gemäß einem der Ansprüche 1 bis 3, wobei R₅ und R₆ unabhängig voneinander aus einem Wasserstoffatom, einem (C₁-C₆)-Alkylrest oder einem (C₃-C₇)-Cycloalkylrest ausgewählt sind.

5. Photochrome Fluorenopyrane mit definierter Dibenzo[b,d]-pyrano-Annellierung gemäß einem der Ansprüche 1 bis 4, welche die allgemeine Formel (I) aufweisen.

6. Photochrome Fluorenopyrane mit definierter Dibenzo[b,d]-pyrano-Annellierung gemäß einem der Ansprüche 1 bis 5, welche die nachfolgende allgemeine Formel (III) aufweisen: 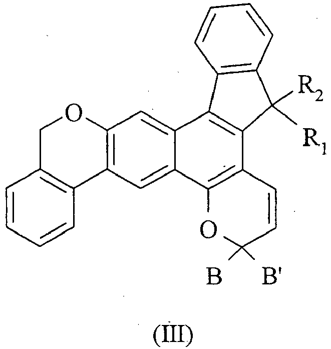 wobei die Reste R₁, R₂, B sowie B' wie vorstehend definiert sind.

7. Photochrome Fluorenopyrane mit definierter Dibenzo[b,d]-pyrano-Annellierung gemäß einem der Ansprüche 1 bis 6, wobei die Reste B und B' unabhängig voneinander aus der Gruppe a), wie vorstehend definiert, ausgewählt sind.

8. Verwendung der photochromen Fluorenopyrane mit definierter Dibenzo[b,d]-pyrano-Annellierung nach einem der Ansprüche 1 bis 7 in und auf Kunststoffmaterialien.

9. Verwendung nach Anspruch 8, wobei das Kunststoffmaterial eine ophthalmische Linse ist.

## Claims

1. Photochromic fluorenopyrans with defined dibenzo[b,d]-pyrano annelation according to the general formula (I) or (II): 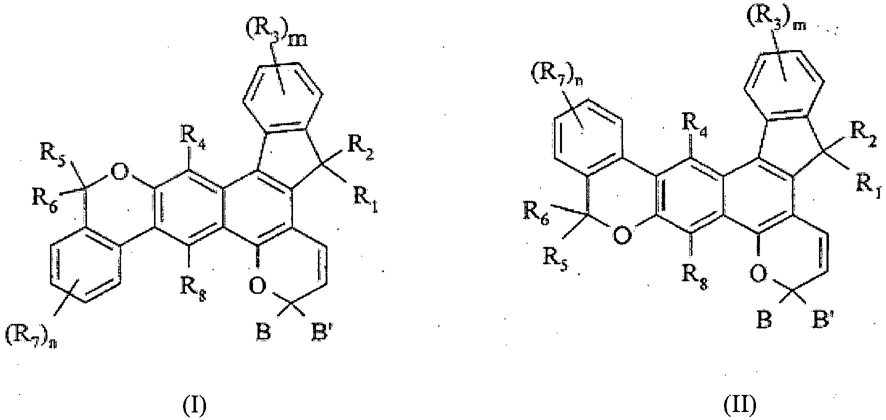
wherein residues R₁, R₂, R₃, R₄, R₅, R₆, R₇ and R₈, respectively independently of one another, represent a substituent selected from the α group consisting of a hydrogen atom, a (C₁-C₆) alkyl residue, a (C₁-C₆) thioalkyl residue, a (C₃-C₇) cycloalkyl residue that can have one or more heteroatoms such as O or S, for example, a (C₁-C₆) alkoxy residue, a hydroxy group, a trifluoromethyl group, bromine, chlorine, fluorine, an un-, mono- or disubstituted phenyl-, phenoxy-, benzyl-, benzyloxy-, naphthyl- or naphthoxy residue, wherein the substituents can in turn be selected from the α group, preferably from (C₁-C₆) alkyl, (C₁-C₆) alkoxy, bromine, chlorine or fluorine;
m and n independently of one another represent a whole number from 1 to 4;
or residues R₁ and R₂ together with the carbon atom bonded to these residues form a 3- to 8-membered carbo- or heterocyclic ring, which possibly carries one or more, preferably one to four, substituents from the α group, but wherein one to three aromatic or heteroaromatic ring systems can also be annelated to this ring, wherein the ring system or systems is or are selected independently from one another from the β group consisting of benzol, naphthalene, phenanthrene, pyridine, quinoline, furan, thiophene, pyrrole, benzofuran, benzothiophene, indole and carbazole, which can in turn be substituted with one or more substituents selected from the α group, and wherein, if two of these substituents carried on the 3- to 8-membered carbo- or heterocyclic ring sit on the same ring carbon atom, these can in turn form a 3- to 8-membered carbo- or heterocyclic ring,
or residues R₅ and R₆ together with the carbon atom bonded to these residues form a 3- to 8-membered carbo- or heterocyclic ring, which possibly carries one or more, preferably one to four, substituents from the α group, but wherein one to three aromatic or heteroaromatic ring systems can also be annelated to this ring, wherein the ring system or systems is or are selected independently from one another from the P group consisting of benzol, naphthalene, phenanthrene, pyridine, quinoline, furan, thiophene, pyrrole, benzofuran, benzothiophene, indole and carbazole, which can in turn be substituted with one or more substituents selected from the α group, and wherein, if two of these substituents carried on the 3- to 8-membered carbo- or heterocyclic ring sit on the same ring carbon atom, these can in turn form a 3- to 8-membered carbo- or heterocyclic ring,
or two adjacent residues R₃ form an annelated benzol ring, which can be un-, mono- or disubstituted, wherein the substituents can in turn be selected from the α group;
or two adjacent residues R₇ form an annelated benzol ring, which can be un-, mono- or disubstituted, wherein the substituents can in turn be selected from the α group;
and B and B', independently of one another, are selected from the following groups a) or b), wherein they are
a) mono-, di- and trisubstituted aryl residues, wherein the aryl residue is phenyl, naphthyl or phenanthryl,
b) unsubstituted, mono- and disubstituted heteroaryl residues, wherein the heteroaryl residue is pyridyl, furanyl, benzofuranyl, thienyl, benzothienyl, 1,2, 3,4-tetrahydrocarbazolyl or julolidinyl,
wherein the substituents of the aryl or heteroaryl residues in a) and b) are those selected from the above-defined α group or from the χ group consisting of amino, mono-(C₁-C₆) alkyl amino, di-(C₁-C₆) alkyl amino, mono- and diphenylamino un-, mono- disubstituted on the phenyl ring, piperidinyl, N-substituted piperazinyl, pyrrolidinyl, imidazolidinyl, pyrazolidinyl, indolinyl, morpholinyl, 2,6-dimethylmorpholinyl, thiomorpholinyl, azacycloheptyl, azacyclooctyl, un-, mono- or disubstituted phenothiazinyl, un-, mono- or disubstituted phenoxazinyl, un-, mono- or disubstituted 1,2,3,4-tetrahydroquinolinyl, un-, mono- or disubstituted 2,3-dihydro-1,4-benzoxazinyl, un-, mono- or disubstituted 1,2,3,4-tetrahydroisoquinolinyl, un-, mono- or disubstituted phenazinyl, un-, mono- or disubstituted carbazolyl, un-, mono- or disubstituted1,2,3,4-tetrahydrocarbazolyl and un-, mono- or disubstituted10,11-dihydrodibenz[b,f]azepinyl, wherein the substituent or substituents, independently of one another, can in turn be selected from (C₁-C₆) alkyl, (C₁-C₆) alkoxy, bromine, chlorine or fluorine;
or wherein two directly adjacent substituents of the aryl or heteroaryl residues in a) and b) represent a V-(CR₈R₉)ₚ-W grouping, wherein p = 1, 2 or 3, residues R₈ and R₉, respectively independently of one another, represent a substituent selected from the α group, and wherein V and W, independently of one another, can be -O-, -S-, -N-(C₁-C₆) alkyl, -NC₆H₅, -CH₂-, -C(CH₃)₂- or -C(C₆H₅)₂-, wherein two or more adjacent CR₈R₉ units of this V-(CR₈R₉)ₚ-W grouping can be part of a benzol ring annelated thereto, which can respectively in turn have one or more substituents selected from the a group, or V and/or W together with the respectively adjacent CR₈R₉ unit represent an annelated benzol ring that can be un-, mono- or disubstituted, wherein the substituents thereof can be selected from the α group.

2. Photochromic fluorenopyrans with defined dibenzo[b,d]-pyrano annelation according to claim 1, wherein residues R₁ and R₂, independently of one another, are selected from a hydrogen atom, a (C₁-C₆) alkyl residue or a (C₃-C₇) cycloalkyl residue, preferably a (C₁-C₆) alkyl residue or a (C₃-C₇) cycloalkyl residue.

3. Photochromic fluorenopyrans with defined dibenzo[b,d]-pyrano annelation according to claim 1, wherein R₁ and R₂ together with the carbon atom bonded to these residues form a 5- to 7-membered carbo- or heterocyclic ring, which possibly carries one or more substituents from the α group.

4. Photochromic fluorenopyrans with defined dibenzo[b,d]-pyrano annelation according to one of claims 1 to 3, wherein R₅ and R₆, independently of one another, are selected from a hydrogen atom, a (C₁-C₆) alkyl residue or a (C₃-C₇) cycloalkyl residue.

5. Photochromic fluorenopyrans with defined dibenzo[b,d]-pyrano annelation according to one of claims 1 to 4, which have the general formula (I).

6. Photochromic fluorenopyrans with defined dibenzo[b,d]-pyrano annelation according to one of claims 1 to 5, which have the following general formula (III): 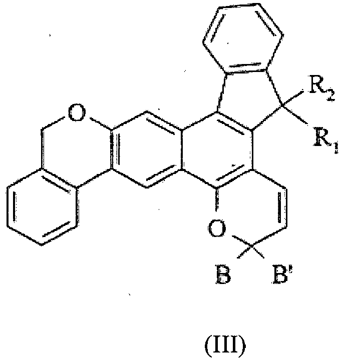 wherein residues R₁, R₂, B and also B' are defined as above.

7. Photochromic fluorenopyrans with defined dibenzo[b,d]-pyrano annelation according to one of claims 1 to 6, wherein residues B and B', independently of one another, are selected from group a), as defined as above.

8. Use of the photochromic fluorenopyrans with defined dibenzo[b,d]-pyrano annelation according to one of claims 1 to 7 in and on plastic materials.

9. Use according to claim 8, wherein the plastic material is an ophthalmic lens.

## Revendications

1. Fluorénopyranes photochromes présentant une annellation dibenzo[b,d]-pyrano définie selon les formules générales (I) ou (II) : 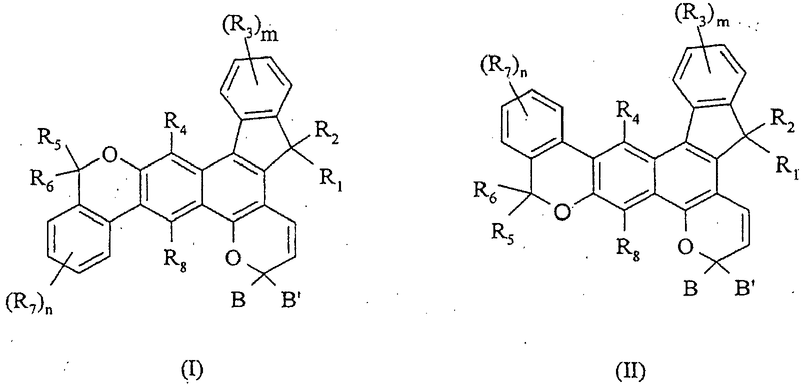
les radicaux R₁, R₂, R₃, R₄, R₅, R₆, R₇ et R₈ représentant respectivement indépendamment les uns des autres un substituant, choisi parmi le groupe α, se composant d'un atome d'hydrogène, d'un radical alkyle en C₁-C₆, un radical thioalkyle en C₁-C₆, un radical cycloalkyle en C₃-C₇, qui peut présenter un ou plusieurs hétéroatomes, par exemple O ou S, un radical alcoxy en C₁-C₆, un groupement hydroxyle, un groupement trifluorométhyle, le brome, le chlore, le fluor, un radical phényle, phénoxy, benzyle, benzyloxy, naphtyle ou naphtoxy non substitué, mono- ou disubstitué, les substituants pouvant être choisis à nouveau parmi le groupe α, de préférence parmi un alkyle en C₁-C₆, un alcoxy en C₁-C₆, le brome, le chlore ou le fluor ;
m et n représentant indépendamment l'un de l'autre un nombre entier allant de 1 à 4,
ou les radicaux R₁ et R₂ formant conjointement avec l'atome de carbone lié à ces radicaux un cycle carbo- ou hétérocyclique de 3 à 8 membres, qui le cas échéant, porte un ou plusieurs substituants, de préférence de un à quatre substituants provenant du groupe α, un à trois systèmes de cycles aromatiques ou hétéroaromatiques pouvant aussi être annelés sur ce cycle, le ou les systèmes de cycle étant choisi ou choisis indépendamment les uns des autres parmi le groupe β se composant de benzène, naphthalène, phénanthrène, pyridine, quinoléine, furane, thiophène, pyrrole, benzofurane, benzothiophène, indole et carbazole, qui peuvent être substitués à nouveau par un ou plusieurs substituants, choisis parmi le groupe α, et si deux de ces substituants portés sur le cycle carbo- ou hétérocycliques de 3 à 8 membres se trouvant sur le même atome de carbone du cycle, ceux-ci peuvent à nouveau former un cycle carbo- ou hétérocyclique de 3 à 8 membres,
ou les radicaux R₅ et R₆ formant conjointement avec l'atome de carbone lié à ces radicaux un cycle carbo- ou hétérocyclique de 3 à 8 membres, qui, le cas échéant, porte un ou plusieurs substituants, de préférence de un à quatre substituants provenant du groupe α, un à trois systèmes de cycles aromatiques ou hétéroaromatiques pouvant aussi être annelés sur ce cycle, le ou les systèmes de cycle étant choisi ou choisis indépendamment les uns des autres parmi le groupe β se composant de benzène, naphthalène, phénanthrène, pyridine, quinoléine, furane, thiophène, pyrrole, benzofurane, benzothiophène, indole et carbazole, qui peuvent être substitués à nouveau par un ou plusieurs substituants, choisis parmi le groupe α, et si deux de ces substituants portés sur le cycle carbo- ou hétérocyclique de 3 à 8 membres se trouvant sur le même atome de carbone du cycle, ceux-ci peuvent à nouveau former un cycle carbo- ou hétérocyclique de 3 à 8 membres,
ou deux radicaux R₃ adjacents formant un cycle de benzène annelé, qui peut être non substitué, mono- ou disubstitué, les substituants pouvant à nouveau être choisis parmi le groupe α ;
ou deux radicaux R₇ adjacents formant un cycle de benzène annelé, qui peut être non substitué, mono- ou disubstitué, les substituants pouvant à nouveau être choisis parmi le groupe α ;
et B et B' étant choisis indépendamment l'un de l'autre parmi un des groupes suivants a) ou b),
a) étant des radicaux aryle mono-, di- et trisubstitués, le radical aryle étant le phényle, le naphtyle ou le phénanthryle ;
b) étant des radicaux hétéroaryle non substitués, mono- et disubstitués, le radical hétéroaryle étant le pyridyle, le furanyle, le benzofuranyle, le thiényle, le benzothiényle, le 1,2,3,4-tétrahydrocarbazolyle ou le julolidinyle,
les substituants des radicaux aryle ou hétéroaryle étant ceux des groupes a) et b), choisis parmi le groupe α défini ci-dessus ou le groupe χ, se composant de amino, mono-(C₁-C₆)-alkylamino, di-(C₁-C₆)-alkylamino, sur le cycle phényle mono- et diphénylamino non substitué, mono- ou disubstitué, pipéridinyle, pipérazinyle n-substitué, pyrrolidinyle, imidazolidinyle, pyrazolidinyle, indolinyle, morpholinyle, 2,6-diméthylemorpholinyle, thiomorpholinyle, azacycloheptyle, azacyclooctyle, phénothiazinyle non substitué, mono- ou disubstitué, phénoxazinyle non substitué, mono- ou disubstitué, 1,2,3,4-tétrahydroquinolinyle non substitué, mono- ou disubstitué, 2,3-dihydro-1,4-benzoxazinyle non substitué, mono- ou disubstitué, 1,2,3,4,-tétrahydroisoquinolinyle non substitué, mono- ou disubstitué, phénazinyle non substitué, mono- ou disubstitué, carbazolyle non substitué, mono- ou disubstitué, 1,2,3,4-tétrahydrocarbazolyle non substitué, mono- ou disubstitué et 10,11-dihydrodibenzo[b,f]azépinyle non substitué, mono- ou disubstitué, le ou les substituants pouvant être choisis indépendamment les uns des autres à nouveau parmi un alkyle en C₁-C₆, un alcoxy en C₁-C₆, le brome, le chlore et le fluor ;
ou deux substituants immédiatement adjacents des radicaux aryle ou hétéroaryle représentant dans a) et b) un groupement V- (CR₈R₉)ₚ-W-, p étant égal à 1, 2 ou 3, les radicaux R₈ et R₉ pouvant représenter respectivement indépendamment l'un de l'autre un substituant, choisi parmi le groupe α, et V et W pouvant être indépendamment l'un de l'autre -O-, -S-,-N-alkyle en C₁-C₆, -N-C₆H₅, -CH₂-, -C(CH₃)₂- ou -C(C₆H₅)₂-, deux ou plusieurs unités CR₈R₉ adjacentes de ce groupement V-(CR₈R₉)ₚ-W- pouvant faire partie d'un cycle de benzène annelé sur celui-ci, lequel peut présenter respectivement à nouveau un ou plusieurs substituants, choisis parmi le groupe α, ou V et/ou W représentent conjointement avec respectivement l'unité CR₈R₉ adjacente un cycle benzène annelé, qui peut être non substitué, mono- ou disubstitué, dont les substituants pouvant être choisis parmi le groupe α.

2. Fluorénopyranes photochromes présentant une annellation dibenzo[b,d]-pyrano définie selon la revendication 1, les radicaux R₁ et R₂ étant choisis indépendamment l'un de l'autre parmi un atome d'hydrogène, un radical alkyle en C₁-C₆ ou un radical cycloalkyle en C₃-C₇, de préférence un radical alkyle en C₁-C₆ ou un radical cycloalkyle en C₃-C₇.

3. Fluorénopyranes photochromes présentant une annellation dibenzo[b,d]-pyrano définie selon la revendication 1, R₁ et R₂ formant conjointement avec l'atome de carbone lié à ces radicaux un cycle carbo-ou hétérocyclique de 5 à 7 membres, qui le cas échéant porte un ou plusieurs substituants provenant du groupe α.

4. Fluorénopyranes photochromes présentant une annellation dibenzo[b,d]-pyrano définie selon l'une quelconque des revendications 1 à 3, R₅ et R₆ étant choisis indépendamment l'un de l'autre parmi un atome d'hydrogène, un radical alkyle en C₁-C₆ ou un radical cycloalkyle en C₃-C₇.

5. Fluorénopyranes photochromes présentant une annellation dibenzo[b,d]-pyrano définie selon l'une quelconque des revendications 1 à 4, lesquelles présentent la formule générale (I).

6. Fluorénopyranes photochromes présentant une annellation dibenzo[b,d]-pyrano définie selon l'une quelconque des revendications 1 à 5, lesquelles présentent la formule générale suivante (III) : 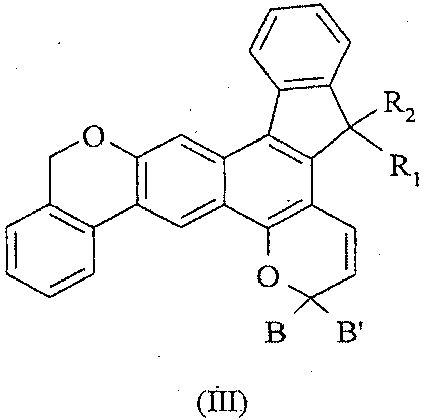 les radicaux R₁, R₂, B ainsi que B' étant définis comme indiqué ci-dessus.

7. Fluorénopyranes photochromes présentant une annellation dibenzo[b,d]-pyrano définie selon l'une quelconque des revendications 1 à 6, les radicaux B et B' étant choisis indépendamment l'un de l'autre parmi le groupe a), comme défini ci-dessus.

8. Utilisation des fluorénopyranes photochromes présentant une annellation dibenzo[b,d]-pyrano définie selon l'une quelconque des revendications 1 à 7 dans et sur des matières plastiques.

9. Utilisation selon la revendication 8, la matière plastique étant une lentille ophtalmique.
